Europäisches Patentamt

European Patent Office

Office européen des brevets

(11) Publication number: **0 193 406 B1**

(12) # EUROPEAN PATENT SPECIFICATION

(45) Date of publication of patent specification: 20.02.91 (51) Int. Cl.⁵: **A61F 2/48**

(21) Application number: 86301429.6

(22) Date of filing: 27.02.86

(54) Device for controlling incontinence.

(30) Priority: 28.02.85 US 706699
28.02.85 US 706700

(43) Date of publication of application:
03.09.86 Bulletin 86/36

(45) Publication of the grant of the patent:
20.02.91 Bulletin 91/08

(84) Designated Contracting States:
DE FR GB IT NL

(56) References cited:
DE-A- 2 811 383
DE-C- 867 126
GB-A- 2 099 304
US-A- 3 768 102
US-A- 4 237 893

(73) Proprietor: MEDTRONIC, INC.
3055 Old Highway Eight P.O. Box 1453
Minneapolis Minnesota 55440(US)

(72) Inventor: Brennen, Kenneth R.
5701 Central Avenue NE
Minneapolis Minnesota 55432(US)
Inventor: Cobian, Kenneth E.
3025 Silver Lake Road
St. Anthony Minnesota 55418(US)
Inventor: Shade, Judith M.
7494 Mariner Drive
Maple Grove Minnesota 55369(US)
Inventor: Cahalan, Patrick T.
10871 133rd Circle
Champlin Minnesota 55316(US)
Inventor: Haeg, Daniel C.
1425 93rd Avenue North
Brooklyn Park Minnesota 55444(US)

(74) Representative: Pett, Christopher Phineas et
al
Frank B. Dehn & Co. European Patent Attor-
neys Imperial House 15-19 Kingsway
London WC2B 6UZ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to devices for controlling incontinence.

Voluntary control over the discharge of bladder contents has long been a serious and distressing problem for persons whose natural urethral valve is no longer capable of completely controlling the outflow of urine from the bladder for such reasons as advanced age, surgery, disease or malformation of the natural urethral valve. Persons with this problem have attempted various means of control or correction which have proved to be either uncomfortable, inconvenient, unsanitary, offensive and/or inadequate.

One method of caring for involuntary bladder emissions consists of the incontinent person wearing a pad of absorbant material in close proximity to the external opening of the urethra to absorb all the fluid that escapes past the malfunctioning natural urethral valve. This method is undesirable in that the wearer is continually confronted with the embarrassment of having to wear a bulky pad of absorbant material that can create skin irritations and offensive odors. A person thus equipped will of necessity feel unduly restricted in their activities ·since they must always be aware of the problems associated with urination, used pad removal and disposal, and pad replacement.

Another method of caring for involuntary urinary emissions has been for the incontinent person to wear a receiving container attached to the leg or suspended from the waist and connected to the external opening of the urethra in such a manner as to allow the urine to flow by the force of gravity into the receiving container. Such a device is disclosed by Snyder, U.S. Patent No. 3,447,536. Not only is such a device cumbersome, but it can be malodorous and has on occasion been known to spill or otherwise accidently discharge its contents.

Neither of the foregoing methods is directed towards solving the underlying problem of the incontinent person. Nor do they attempt to return the evacuation of bladder contents to the completely voluntary control of the afflicted person.

Beliveau et al, U.S. Patent No. 3,372,694, proposed a device that is inserted up the urethra until a retainer portion resides inside the bladder and an appendage thereto or plug passes out of the bladder into the urethra such that the plug resides within the confines of the urethral sphincter muscle. The appendage or plug portion of their device that passes out through the urethral sphincter muscle is smaller in diameter than the internal diameter of the urethra but of sufficient diameter to form a fluid barrier when the urethral sphincter muscle is closed about it. Their invention is adaptable only for those persons who are able to partially close the urethral sphincter muscle sufficiently to completely restrict the urethra when the plug is located within the confines of the muscle and thus overcome incontinence of urination. Their invention would not be usable in a person who was unable to exercise any form of voluntary control over the urethral sphincter muscle or in a person whose urethral sphincter muscle has been damaged by disease such that the plug portion would not complete the seal when acted upon by the muscle. Nor would it be operable in a person whose urethral sphincter muscle has been surgically removed for one reason or another.

The device of the present invention does not require the use of any external appliances as disclosed by Snyder or the adaption of other voluntary muscles to the task of supplying motive power to a urethral closure device located externally of the urethra as disclosed by Berry, U.S. Patent No. 3,066,667.

U.S. Patent No. 4,480,642 to Vladimir A. Stoy et al entitled "Dilation Device for the Cervix" relates to a cervical canal swelling device having an essentially cylindrically shaped stem formed in major portion thereof of a dehydrated hydrogel of a certain swelling capacity and enforced uniaxial deformation. The cervical dilator of Stoy et al is intended to overcome the disadvantages of cervical dilators made of natural materials. Moreover, in contrast with the presention invention, Stoy et al contemplate the implementation of a dehydrated or dried hydrogel in their cervical dilator.

German DE-C-867126 discloses a form of urethral plug which is carefully engineered to be at least partially resilient. The plug is adapted to be inserted into the uretha with the help of a pin which stretches a wider, flexible portion of the plug into a cylindrical shape for insertion, but when the pin is removed, the plug readopts a widened profile thus producing a seal. We are not aware that such a proposal has ever been employed, or that is has proved practical.

German DE-A-2811383 discloses a stoma closure in the form of a swellable hydrogel supported within a flexible body-fluid-porous container. Such a device has little rigidity and is of a size suitable for stoma openings, these being generally much longer than urethras.

The present invention provides a device which assists the urethral sphincter muscle in effecting urethra closure and utilizes only the normal muscles used in urination to create sufficient pressure upon the bladder contents.

The present invention provides an incontinence device or urethral plug which is generally elongate, cylindrical or bullet-shaped. The device of this invention is generally of urethral diameter and is, therefore, substantially longer than it is wide. At least a portion of the device comprises a hydrophilic, body fluid swellable hydrogel most desirably comprising, before fluid absorption, at least about 40% by weight water which, after insertion into the urethra and exposure to fluids therein, e.g. urine or mucous, controllably swells or expands to cooperate with the walls of the urethra to provide a soft, comfortable, conformable, flexible, non-irritating fluid restrictive fit thereby controlling involuntary urination or incontinence. Such a portion of hydrogel is disposed about a substantially non-swellable flexible core.

In a preferred embodiment, the present device includes removal means which enable convenient removal of the device from the urethra when bladder evacuation is desired and stopper means to prevent the device from entering the bladder. In yet another embodiment of this invention, the preferred device includes a flexible cuff or flap which is preferably disposed adjacent the zone of the hydrophilic swellable gel and, prior to insertion of the device, overlies or surrounds at least a portion of the gel in a first position along the body of the device. After the device is inserted into the urethra e.g., by means of an applicator, the cuff is released from its first position and opens to a second position angularly disposed with respect to the axis of the device to provide further restriction to bladder entry and additional fluid retention. The cuff may be intentionally constructed so that it has a tendency to spring from its first position to its second position. Alternatively it may be constructed so that it moves or opens more slowly to its second position as the hydrogel expands with the uptake of body fluids.

Urinary incontinence can be controlled by the steps of introducing into the urethra, preferably to about the mid segment or mid region of the urethra, a urinary incontinence device of the invention, permitting the fluid swellable gel to be exposed to body fluids and thereby to expand controllably to provide a substantially fluid restrictive fit with the walls of the urethra and thereby prevent involuntary external urination. The device may be removed from the urethra when voluntary bladder evacuation is desired. In a preferred practice of this method, female urinary incontinence is controlled. In another practice of this method, a device according to the invention in which the hydrophilic gel comprises about 40% by weight water, is inserted into the male urinary tract, e.g., the penis. The device is permitted to absorb body fluid, e.g., urine, and thereby to expand controllably to provide a restrictive fit with the walls of the penile urethra. When the wearer feels the need to urinate, the flexible, compressible device is removed, and voluntary urination proceeds.

Aspects of the invention may be more readily understood by reference to the attached drawings which are by way of example only and wherein like features are designated by like numerals throughout.

In the drawings,

Fig. 1 is a perspective view of a device of the present invention in its applicator prior to insertion;

Fig. 2 is a cross-sectional view of one embodiment of the device of the present invention;

Fig. 3 is a cross-sectional view of a second embodiment of the device of the present invention;

Fig. 4 is a sectioned view of the embodiment of the present invention depicted in Fig. 3 taken along line 4-4;

Fig. 5a is a cross-sectional view of the embodiment of the invention depicted in Fig. 3 taken along 5-5 before swelling of the hydrogel;

Fig. 5b is a cross-sectional view of the device depicted in Fig. 3 taken along line 5-5 after swelling of the hydrogel;

Fig. 6 is a cross-sectional view of another embodiment of the device of the present invention;

Fig. 7 is a cross-sectional view of yet a further embodiment of the device of the present invention;

Fig. 8 is a cross-sectional view of the embodiment of the invention depicted in Fig. 7 taken along line 8-8;

Figs. 9a and 9b are sectioned view of the embodiment of the invention depicted in Fig. 7 taken along line 9-9, 9b indicating the change in cross-section by virtue of the uptake of fluid by the swellable gel.

Fig. 10 is a sectioned view of the applicator assembly and device pictured in Fig. 1.

## DETAILED DESCRIPTION OF THE INVENTION

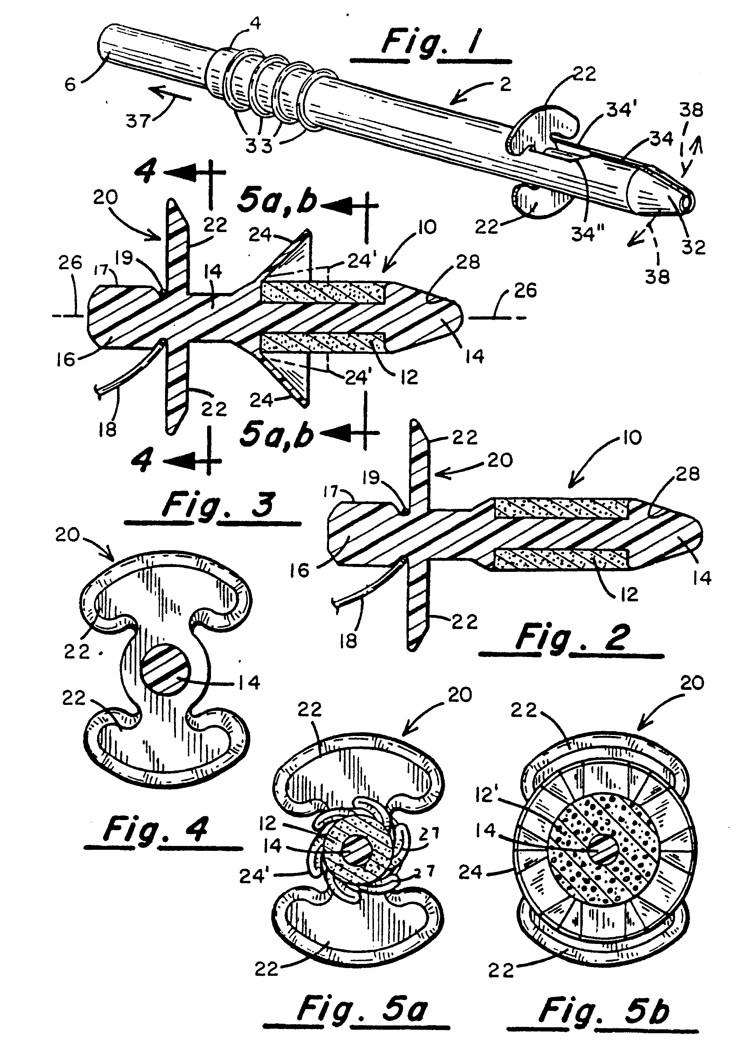

Fig. 1 is a perspective view of an embodiment of an incontinence device of the invention inside an applicator 2. Several embodiments of the device are more completely described below.

Referring now in particular to Figs. 2, 3, 6 and 7, there is shown the generally elongate, cylindrical, bullet-shaped incontinence device or plug 10 of the invention. As indicated above, at least a portion of the device 10 comprises a body fluid swellable, hydrophilic gel (hydrogel) 12 comprising, before swelling upon exposure to body fluid, at least about 40% by weight water up to a maximum of about 80 weight percent water. In a preferred practice, the hydrogel employed herein comprises at least about 50 weight percent water. It is within the contemplation of this invention that the entire device comprise such a gel. In a preferred practice of this invention, plug 10 includes a non-swellable, flexible, elastomeric core 14 along which there is disposed the cylindrical zone or region of body fluid swellable hydrophilic gel 12.

The device 10 optionally includes a removal means, which in this depiction, comprises a bulb 16 and, for purposes of emergency removal, an optional string 18 disposed about the neck 19 of the bulb 16. Bulb 16, as shown, has flattened sides 17 which permit the device to be more easily ejected from the urethral insertion tube discussed below. Bulb 16 could be substantially spherical or other suitable shape. Removal means 16, 18 is intended to be manually employed, and therefore bulb 16 is merely one possible embodiment of a number of possible structures which could provide a convenient grip for removal of the device from the urethra with the fingers.

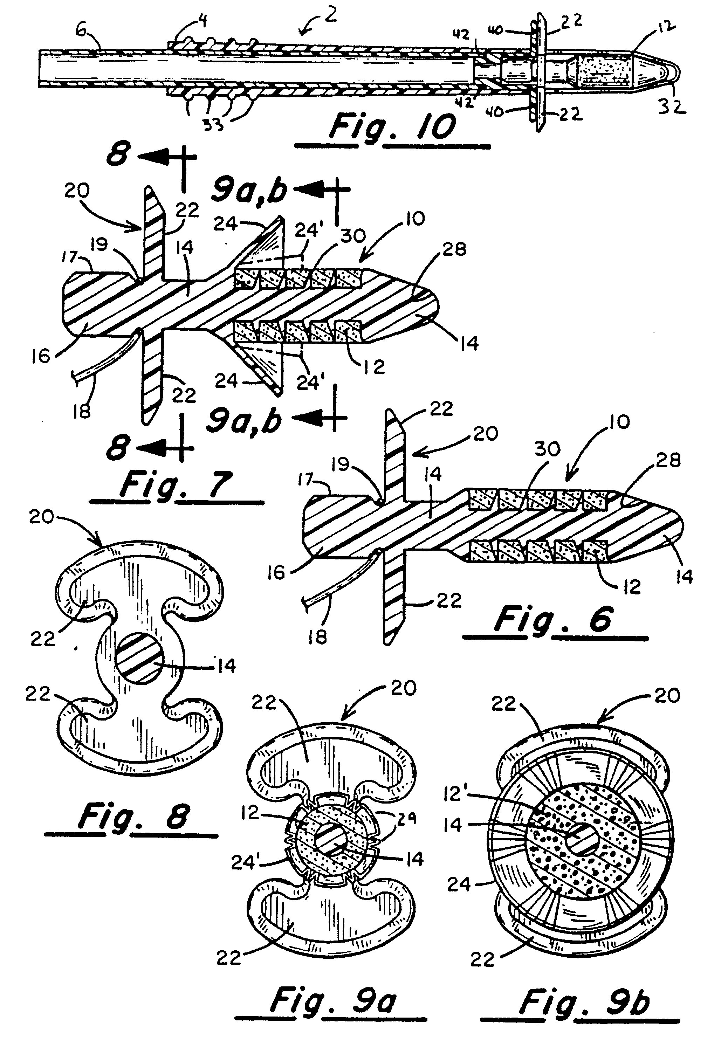

As is most clearly illustrated in Fig. 4, this embodiment of the invention includes a stopper means 20 which comprises radially disposed, kidney-shaped or hemispherical flanges 22 located on opposite sides of the device. The two flanges 22, as shown, provide a figure "8" stop. Radial flanges 22 of the figure "8" stop are intended to cooperate with the external meatus of the urethra to prevent the device from migrating into the bladder and thus are fairly longitudinally rigid. The embodiments of the invention depicted in Figs. 3 and 7 also indicate the presence of a preferred flap 24. Flap 24 is longitudinally flexible and intended to have a first position substantially axially disposed adjacent the shaft of the device and parallel to the axis of the device shown by line 26-26 (it is depicted in phantom 24' in Figs. 3 and 7). Flap 24 has a second position angularly disposed with respect to the axis 26 of the device. As is most clearly seen in Figs. 5a and 5b, flap 24 is umbrella-like, extending (in this embodiment) radially 360° about the shaft or body of the device. While the precise angle between flap 24 and the axis 26 of the device is not critical, it should generally fall in the range of about 10° - 60°, preferably about 30° - 50° and most preferably about 45°. In the embodiment of Fig. 5a, flap 24' is shown with overlapping folded sections 27. In the embodiment of Fig. 9a, the flap is comprised of a plurality of fluted gussets 29. As is shown in Figs. 2, 3, 6 and 7, the device has a tapered end portion 28 to permit ease of insertion into the urethral tract.

Figs. 5a and 9a illustrate flap 24' disposed in a first position adjacent and overlapping unswollen hydrogel 12 in its first position (e.g., as the device would appear in the applicator before insertion). Figs. 5b and 9b depict flap 24 in a second, angularly disposed position, such as it would appear after insertion into the urethra and the applicator withdrawn. Figs. 5b and 9b also show the hydrogel 12' in its swollen or fluid-absorbed state. To be effective and comfortably cooperate with the urethral tract, the hydrogel 12, 12' should desirably swell approximately 10% to 100% of its original diameter to assist in the prevention of incontinence. The comfort of the patient determines the extent of swell to be utilized. As is more clearly explained below, the extent of swell is controllable by means of selection of the gel chemistry, the length of time to which the gel is exposed to body fluids and the pressure exerted on the device by the surrounding urethral tissue.

Figs. 6-9b illustrate another embodiment of the invention wherein fluid swellable gel 12 is held adjacent central core 14 by gel retention means 30. In this embodiment, gel retention means 30 comprise radial ribs. Other gel retention means which tend to anchor the gel 12 adjacent the elongate body of the device will be obvious to one skilled in the art.

Fig. 1 is a perspective view of an incontinence device of the invention 10 located within an applicator 2. As shown, applicator 2 includes an exterior introducer sleeve 4, an interior insert tube 6 which abuts bulb 16 and the back side of the two flanges 22 of the figure "8" stop of the device 10. Introducer sleeve 4 has a tapered end 32 which is adapted for urethral insertion to about the mid region of the urethra. On the end of the introducer opposite the tapered end 32 is a grip means 33, e.g., a plurality of ridges or rings. On the tapered end of the introducer 32, there is a longitudinal slit or opening 34 which has a first narrower width on its portion which intersects the tapered end 32 of the applicator 2 and a wider slit portion 34' which permits the exterior introducer sleeve 4 to be withdrawn from around the device 10 by means insert tube 6. During insertion, the thumb is held against insert tube 6 and the fingers are placed around grip rings 33. The device in the applicator 2 is then inserted into the urethra. After insertion of the applicator 2 into the urethra, the insert tube 6 is held substantially stationary, and the exterior introducer sleeve 4 is withdrawn from around the incontinence device in the direction of arrow 37. This causes the device 10 to be ejected from the applicator 2 with the two tapered end portions 32 of the applicator 2 separated at slit 34 opening in the direction indicated by arrows 38. In this manner, minimal forward thrust is applied to the device thus reducing the likelihood that the device would be inserted too far into the urethral tract. It is to be noted that narrow slit portion 34 and wider slit portion 34' are connected by means of an angular (i.e., "V" shaped) slit 34". The angle of the "V" shaped slit determines the rapidity with which tapered portions of the applicator 32 are separated during delivery of the incontinence device. Thus the angle with respect to the axis of the device (and the applicator) at which wide slit portion 34" is blended to narrow slit portion 34 may determine

EP 0 193 406 B1

the comfort with which the device is inserted.

After the incontinence device 10 is delivered from the applicator 2 into the urethra, optional flap 24, being flexible, tends to open up from its first position 24' to its second position 24. (as shown in Figs. 3 and 7). At this point, the swellable gel 12 begins to absorb body fluid and expand. Within a time period as short as 5 to 10 minutes, the gel 12 will have expanded to 5% - 20% of its original diameter and, in cooperation with optional flap 24 will provide enhanced control of urinary incontinence. It should be noted that the force of the swelling hydrogel 12 could be used to unfold flap 24 from its first to its second position. As a function of time, the change in outside diameter of the device at 37°C in deionized water of a typical hydrogel would be as follows:

| Time, Min. | % Change in Outside Diameter |
|---|---|
| 5 | 3-25 |
| 10 | 4-32 |
| 30 | 10-48 |
| 60 | 20-62 |
| 240 | 45-116 |

Fig. 10 is a cross-section view of the assembly shown in perspective in Fig. 1. In Fig. 10, applicator 2 is shown to comprise exterior introducer sleeve for an interior insert tube 6. Also shown is the tapered end 32 of introducer sleeve 4 which is adapted for urethral insertion. Grip means 33 (e.g., ridges or rings) are also shown. Of particular note in Fig. 10 is optional stop or insert tube stop 40. During insertion of the device into the urethra, it is particularly important that the device be deposited at the desired point of insertion without being thrust further up the urethral canal. Stop 40 prevents the use of insert tube 6 to force the device 10 further up the urethral canal during insertion. This is accomplished when interior insert tube 6 abuts stop 40 at intersection 42. From that point on during the insertion process, exterior introducer sleeve 4 is basically withdrawn from around the incontinence device 10. Thus the tendency for the device to be thrust or driven further up the urethral canal is restricted.

As discussed above, the device of the present invention contemplates, in a preferred embodiment, a zone or region of a hydrophilic controllably swellable gel. The gel utilized in the present invention must be controllably swellable so that when exposed to urethral bodily fluid, it does not expand to the point where the resulting pressure on the urethra causes pain or other unnecessary discomfort. Further, the gel of the present invention should desirably swell fairly quickly so that incontinence is actively controlled. Lastly, the composition in the present invention must be flexible for comfort, stable in the bodily fluids to which they are exposed and capable of being sufficiently compliant so that when it is desired to evacuate the bladder, the incontinence device can be safely removed without injury to sensitive internal body passages. These characteristics have been found in hydrogels of various hydrogel concentration (i.e., solids content) but which generally contain at least about 40% by weight water and preferably contain at least about 50% by weight water.

With the above constraints, there are likely to be many possible controllably swellable compositions which could be employed in the present invention. A preferred composition for utilization herein generally comprises a copolymer of acrylamide (AA), the sodium salt of 2-acrylamido-2-methyl propanesulfonic acid (NaAMPS) and N,N'-methylenebisacrylamide (MBA) crosslinker.

While N,N'-methylenebisacrylamide is a preferred crosslinker for utilization in the gel, other crosslinkers include N,N' hexamethylenebisacrylamide, N,N'-diallyltartardiamide, 2, 2-bis(acrylamido)-propane sulfonic acid, or an olefinic agarose derivative polyacrylamide gel crosslinker, commercially available from FMC Corp. under the trade designation AcrylAid™.

The fluid-swellable material may be produced by mixing the desired amounts of the monomers with a suitable catalyst such as ammonium persulfate and distilled water. The entire mixture of monomers, catalyst and water then is heated to a temperature of approximately 50°C to effect polymerization. Illustrative composition ranges of a preferred swellable gel are as follows:

| Constituent | Composition Range, Wt% |
|---|---|
| Acrylamide (AA) | 20-50 |
| NaAMPS | 0-20 |
| MBA | 0.0-0.016 |
| Catalyst | 0.04-0.08 |
| Water, distilled | Balance |

It is likely that the individual materials of a preferred composition discussed above would be commercially available from a number of chemical companies. However, the particular materials employed were AA available from Eastman Kodak Company, NaAMPS commercially available from Lubrizol Chemical Company under the trade designation "Lubrizol 2405" (a 50:50 solution in water of the sodium salt of the AMPS monomer), MBA commercially available from Aldrich Chemical Company and ammonium persulfate catalyst, also commercially available from Aldrich Chemical Company. In instances where the AMPS sodium salt (Lubrizol 2405) is employed, a 50% aqueous solution was neutralized to a pH of about 7 using AMPS acid powder (Lubrizol 2404) prior to polymerization of the hydrophilic gel.

Alternative to the above-described hydrophilic gels, it is within the contemplation of the present invention that an interpenetrating polymer network (IPN) be employed based upon crosslinked polyacrylamide or polyacrylamide/NaAMPS copolymer systems. IPN's would generally employ one or more of the following polymer swelling agents: Rheothik 80-11S (the sodium salt of the AMPS polymer), Good-rite K-732 polyacrylic acid, Good-rite K-732 sodium polyacrylate or polyethylene glycol. Such IPN systems may also contain polar non-polymeric swelling agents such as glycerol or sorbitol. An interpenetrating polymer network is to be distinguished from the above described copolymer produced from three separate monomers in that polymerization is carried out within an already established polymer network.

Another class of materials which appear to be operable in the present invention includes thermally processable gels based upon polyvinyl alcohol (PVA). Illustrative of this variety of body-fluid absorptive or swellable material is a composition having about 16-20% PVA, about 10-30% by weight polar plasticizer, about 1-6% by weight sodium Rheothik 80-11S and the remainder distilled water. Examples of polar plasticizers, as above, include glycerol, sorbitol, polyethylene glycol (molecular weight 200-600) alone or in combination. Additional hydrophilic polymers included Rheothik 80-11S (Henkel Company), hydroxypropyl guar commercially available from Henkel Company under the trade designation Galactosol starch graft polymer (SGP) also commercially available from Biosorb and other hydrocolloid gums and resins well known to one skilled in this art. It is believed that the basic characteristics of the material have been adequately described. Any material having the requisite properties of being capable of swelling, resisting dissolution and in body fluid, having the requisite liquid content, and having sufficient cohesive strength so as not to be unstable in the environments contemplated is within the teaching of the present invention. A specific PVA-based hydrophilic gel contemplated by the present invention would have the following composition:

| Ingredient | % By Weight |
|---|---|
| PVA | 18 |
| Rheothik (Na) | 2 |
| Sorbitol | 10 |
| Glycerol | 15 |
| Water, distilled | 55 |

In a preferred embodiment of the present invention, the female urinary incontinence device has a zone or region, generally annular, of the one of the above-discussed hydrogels, disposed on a non-swellable, flexible, elastomeric core 14. Many moldable, soft, flexible, elastomeric materials may be utilized in core 14. However, particularly preferred materials include C-Flex 35A, C-Flex 35A-radiopaque (BaSO₄), Silastic Q7-4840 A/B Medical Grade Liquid Silicone Rubber (Dow Corning), Kraton G-7705, Kraton D-3226 and Kraton D-2104 (Shell Chemical Company) Silastic Q7-4635 and Silastic Q7-4735 medical grade ETR elastomers. The "C-Flex " materials which are available from Concept Polymer Technologies, Inc. in Clearwater, Florida are hydrocarbon block copolymers with dispersed polysiloxane, cf, U.S. Patent No. 4,386,179. The "C-Flex" materials have different numerals indicating the hardness of the various blends. Radiopaque versions of the "C-Flex" 35A, 50A and 70A are commercially available from Concept Polymer Technologies, Inc. Radiopacity is a desirable preferred attribute of a material employed in the elastomeric core so that should it be necessary, x-rays could be used to locate the device. "Kraton" materials are styrene/ethylene/butylene/styrene block copolymers commercially available from Shell Chemicals. The Kraton and C-Flex materials are thermoplastic elastomers while the Silastic "materials" are thermoset (i.e., crosslinked) polymers. Thus it is within the contemplation of this invention that both thermoset and thermoplastic core materials be employed. One of ordinary skill in the requisite art will recognize that there are many materials which exhibit a suitable balance of the mechanical properties of softness, compliance, flexibility, amenability to blending radiopaque additives and adequate toughness and strength for device removal.

The present invention will now be further illustrated by means of several examples which should be understood not to be limiting.

Table I presents Examples 1-4 which are four specific examples of hydrogel formulations synthesized in accordance with conventional free radical polymerization techniques. Examples 1 and 2 represent standard MBA crosslinked polyacrylamide/NaAMPS copolymer hydrogel systems, whereas Examples 3 and 4 illustrate interpenetrating polymer network (IPN) systems also based on MBA crosslinked polyacrylamide/NaAMPS. The polymerization of IPN hydrogel systems are carried out in the same manner as non-IPN hydrogel systems. In the later Examples (3 and 4), the IPN systems are provided by the hydrophilic NaAMPS polymer (Rheothik 80-11S). When synthesizing these hydrogels, care should desirably be taken to eliminate oxygen as much as possible by either purging with nitrogen (or other inert gas) or degassing under vacuum. Careful control of temperatures in the mixing vessel and molds are maintained during the procedure, as well as control over the solution pH (6.0-8.0), dissolved oxygen content (1 < ppm) and level of vacuum (5mm < Hg pressure). Oxygen should most desirably be excluded from the system in order to achieve a hydrogel system of sufficiently high molecular weight and low residual monomer content. High polymer molecular weight and low residual monomer content (i.e., a high degree of polymerization) are necessary to produce material with adequate resistance to gamma radiation encountered during the preferred step of device sterilization.

## TABLE I.

### HYDROGEL FORMULATIONS

|  |  | Composition (Weight Percent) | | | |
|  |  | Example | | | |
| Constituent | Description | 1 | 2 | 3(IPN) | 4(IPN) |
| AA | Monomer | 37.7 | 35.7 | 35.6 | 33.6 |
| NaAMPS* (Lubrizol 2405/ Lubrizol) | Monomer | 2.50 | 4.96 | 4.96 | 7.40 |
| MBA | Monomer/Cross- linker | 0.005 | 0.005 | 0.005 | 0.005 |
| NaAMPS Polymer (Rheothik 80-11S/Henkel) | Hydrophilic polymer for interpenetra- ting network | ------ | ------ | 0.30 | 0.30 |
| Ammonium Persulfate | Catalyst | 0.04 | 0.04 | 0.04 | 0.04 |
| Water, distilled | Solvent | Balance | Balance | Balance | Balance |

*Lubrizol 2405, a 50:50 solution of NaAMPS monomer in water, was pre-neutralized to a pH of 7.05 with Lubrizol 2404 powder prior to formulating. Lubrizol 2404 is the acid version of the AMPS monomer.

The polymerization procedure for synthesizing approximately 400 grams of the hydrogel formulations in Examples 1-4 was as follows:

A 40 weight percent acrylamide (AA) solution was prepared by dissolving 152 grams of AA powder in 228 grams of distilled water. To this solution, 20 grams of NaAMPS solution (Lubrizol 2405 solution pre-neutralized to a pH of 7.05 with Lubrizol 2404 acid AMPS monomer) and 1.3 ml of a 1.6 weight percent MBA solution in distilled water. This solution was transferred into a 1 l reaction kettle (resin kettle) which was equipped with a magnetic stir bar, thermometer, vacuum and nitrogen ports and a pressure equalizing addition funnel for adding catalyst solution. The resin kettle was seated in a heating mantle equipped with a thermosensor temperature controller for precise temperature control. The reaction kettle was connected to a line of molds connected in series using silicone rubber tubing. This mold line was equipped with a peristaltic pump for transferring the catalyzed monomer solution to the molds, a vacuum gauge (Sargant-Welch Tube Model 1515A) and a dry ice moisture trap.

A fresh 8 weight percent catalyst solution of $(NH_4)_2S_2O_8$ in distilled water was prepared. A 2.0 ml aliquote of the catalyst solution was transferred into the pressure equalizing addition funnel which was connected to the cover of the reaction kettle. The monomer and catalyst solutions were evacuated/degassed separately for about 20 minutes. The system pressure (i.e., reaction kettle, connecting tubes and molds) at that time was 2.0 mm Hg. The evacuated/degassed catalyst solution was drained into the evacuated/degassed monomer solution with continuous mixing. After mixing 2-3 minutes, the vacuum was turned off, and the resin kettle, molds and connecting lines were blanketed with nitrogen and the catalyzed monomer solution pumped into the molds. For hydrogel characterization studies, these molds consisted of glass tubes 0.200 and/or 0.237 inches in diameter. When all the molds were filled, they were sealed off and placed in a water bath for 60 minutes to effect the polymerization reaction (cure). The temperature of the water bath while curing was 48-52°C.

The temperature of the reaction kettle was maintained at 15±1°C while the monomer solution was being mixed and evacuated. The pH of the catalyzed monomer solution was 6.59 (determined by an Orion Model 610A digital meter equipped with an Orion combination pH 91-05 probe). The dissolved oxygen content of the catalyzed monomer solution after evacuation/degassing was 0.6 ppm (determined by a YSI Model 54A Oxygen Meter/Probe).

Upon removal from glass tubular molds, the resulting cylindrical hydrogels were characterized before and after gamma sterilization at irridation dosages of 2.5-3.5 Mrad. Table II presents typical swell properties for the hydrogel formulations in Examples 1, 2, 3 and 4 carried out on cylindrical test specimens 0.237 inch diameter by 0.25 inch length. Table III presents typical mechanical properties of the hydrogel formulations in Examples 1, 2, 3 and 4.

## TABLE II.

### TYPICAL SWELL PROPERTIES OF HYDROGEL
### CYLINDRICAL TEST SPECIMENS IN DE-IONIZED
### WATER AT 37°C*

| Formulation | Test Condition** | Immersion Time (Minutes) | | | |
| | | 5 | | 30 | |
| | | Change In | | Change In | |
| | | O.D.,% | Wt.,% | O.D.,% | Wt.,% |
|---|---|---|---|---|---|
| Example 1 | Control | 9.4 | 35.3 | 28.8 | 110 |
| | Gamma Sterilized | 13.9 | 39.8 | 32.7 | 109 |
| Example 2 | Control | 15.0 | 55.3 | 41.4 | 176 |
| | Gamma Sterilized | 22.0 | 58.0 | 50.4 | 194 |
| Example 3 | Control | ---- | ---- | ---- | ---- |
| | Gamma Sterilized | 22.6 | 57.3 | 43.7 | 175 |
| Example 4 | Control | ---- | ---- | ---- | ---- |
| | Gamma Sterilized | 24.5 | 75.7 | 55.5 | 237 |

*Test specimen size 0.237 inch diameter X 0.25 inch length.

**Gamma sterilization was carried out at dosages of 2.5-3.5 Mrad.

## TABLE II. Cont.
## TYPICAL SWELL PROPERTIES OF HYDROGEL
## CYLINDRICAL TEST SPECIMENS IN DE-IONIZED
## WATER AT 37°C*

| Formulation | Test Condition** | Immersion Time (Minutes) | | | |
|---|---|---|---|---|---|
| | | 60 | | 240 | |
| | | Change In | | Change In | |
| | | O.D., % | Wt., % | O.D., % | Wt., % |
| Example 1 | Control | 40.5 | 219 | 69.9 | 469 |
| | Gamma Sterilized | 41.4 | 162 | 66.8 | 354 |
| Example 2 | Control | 57.2 | 290 | 86.2 | 674 |
| | Gamma Sterilized | 65.4 | 290 | 105 | 754 |
| Example 3 | Control | ---- | ---- | ---- | ---- |
| | Gamma Sterilized | 65.0 | 282 | 104 | 780 |
| Example 4 | Control | ---- | ---- | ---- | ---- |
| | Gamma Sterilized | 65.0 | 368 | 123 | 1020 |

*Test specimen size 0.237 inch diameter X 0.25 inch length.

**Gamma sterilization was carried out at dosages of 2.5-3.5 Mrad.

TABLE III.

TYPICAL MECHANICAL PROPERTIES OF

HYDROGEL CYLINDRICAL TEST

SPECIMENS AT AMBIENT CONDITIONS

(CROSS-HEAD SPEED 5 INCHES/MINUTE)

| Formulation | Test Condition* | Ultimate Tensile Strength (psi) | Ultimate Elonga- tion(%) | Secant Modulus at 100% Elongation (psi) |
|---|---|---|---|---|
| Example 1 | Control | 30.8 | 1500 | 10.6 |
| | Gamma Sterilized | 22.9 | 380 | 11.2 |
| Example 2 | Control | 32.3 | 1400 | 8.0 |
| | Gamma Sterilized | 13.2 | 220 | 8.4 |
| Example 3 | Control | 28.1 | 1560 | 8.3 |
| | Gamma Sterilized | 24.7 | 400 | 10.3 |
| Example 4 | Control | 29.0 | 1330 | 7.6 |
| | Gamma Sterilized | 14.3 | 254 | 8.9 |

*Gamma sterilization was carried out at dosages of 2.5-3.5 Mrad. Test specimens were cylindrical gels 0.200 inches in diameter x 2 inches in length (gauge length 1.0 inches).

12

## EXAMPLES 5-6

Other IPN formulations contemplated include:

| Constituent | Description | Compositional Range (Weight %) |
|---|---|---|
| I.NaAMPS IPN Polymer Systems With Higher Levels of NaAMPS AND Rheothik* | | |
| AA | Monomer | 20-50 |
| NaAMPS | Monomer | 1.0-15.0 |
| MBA | Crosslinker | 0.0-0.016 |
| Rheothik 80-11S | NaAMPS Polymer for interpenetrating network | 0.10-5.0 |
| Ammonium persulfate | Catalyst | 0.04-0.08 |
| Water, distilled | Solvent | Balance |

and

| Constituent | Description | Compositional Range (Weight %) |
|---|---|---|
| II.Acrylic Acid IPN Polymer Systems | | |
| AA | Monomer | 20-50 |
| NaAMPS | Monomer | 1.0-15.0 |
| MBA | Crosslinker | 0.0-0.016 |
| Acrylic Acid* Polymer | Polymer for interpenetrating network | 0.10-20.0 |
| Ammonium persulfate | Catalyst | 0.04-.08 |
| Water, distilled | Solvent | Balance |

*Good-rite K-732 polyacrylic acid (50% total solids solution) - B.F. Goodrich

Good-rite K-739 sodium polyacrylate powder - B.F. Goodrich

Hydrogels synthesized according to Examples 1-4 were disposed about an incontinence device as depicted in one of Figs. 2 and 6. The length of the incontinence device from its tapered end 28 to the flat portion of the stopper 20 was approximately 1.85 cm (0.73 inches). The diameter of the device and the approximate outside diameter of the gel was 6.6 mm (0.260 inches). While only a particular set of dimensions has been described, it is expected that in the practice of the present invention, a physician will have available a range of devices having various outside diameters to be fit the diameter of the particular patient's urethra. Contemplated sizes range from diameters of 4.6 mm (0.180 inches) to 6.6 mm (0.260 inches). Of great importance in the construction of the device is the distance from stopper means 20 to the hydrogel 12. This distance is determined by the length of the patient's urethra, as well as the urethral anatomical structure (i.e., shape, mechanical properties of urethral tissue and surrounding tissue external to

13

the urethra). The device should be inserted to a site where it is securely placed within the urethra beyond the relatively non-distensible tissue in the region of the meatus, but not so far up the urethra that it may enter the bladder creating conditions favorable for infections and making its removal difficult. This dimension, i.e., the dimension from the face of stopper 20 to the edge of hydrogel 12, generally should fall in the range of approximately 2-5mm (0.079 - 0.197 inches). With the above understanding of the constraints which must be met, some amount of fitting of this distance may be necessary.

One of ordinary skill in the female urinary incontinence device art will recognize there are many possible variations and alternative embodiments of the present invention. These alternative embodiments are to be included within the scope of the present invention as set forth in the claims which follow.

## Claims

1. An incontinence device (10) adapted for insertion into and, when desired, removal from a urethra which includes a portion of hydrophilic, body fluid-swellable hydrogel (12) of substantially urethral dimensions disposed about a substantially non-swellable flexible core (14), which hydrogel, when the device is inserted in use into and exposed to fluids in the urethral tract, is adapted to swell to provide a restrictive fit against the walls of the urethral tract thereby inhibiting or controlling incontinence.

2. A device as claimed in claim 1 wherein the hydrophilic gel comprises at least about 40% by weight water.

3. A device as claimed in claim 1 or claim 2 wherein the hydrogel portion is a belt circumferentially disposed about the core.

4. A device as claimed in any of claims 1 to 3 which further includes stopper means (20) adjacent one end thereof to prevent insertion of the device into the bladder.

5. A device as claimed in claim 4 wherein the stopper means (20) and the swellable gel portion (12) are located adjacent opposite ends of the device.

6. A device as claimed in any of claims 1 to 5 wherein the core includes a tapered end (28) adapted to permit insertion of the device (10) into the urethra, the opposite end thereof including removal means (33) to permit manual removal of the device from the urethra, the hydrogel being circumferentially disposed about the core (14) between the tapered end and the removal means.

7. A device as claimed in any of claims 1 to 6 wherein the hydrophilic gel comprises
   a) at least about 40% by weight water;
   b) 20-50 weight percent polyacrylamide;
   c) 0-20% by weight sodium salt of 2-acrylamido-2-methyl propanesulfonic acid; and
   d) a crosslinker.

8. A device as claimed in any of claims 1 to 6 wherein the hydrogel comprises an interpenetrating polymer network (IPN) including polyacrylamide and a polymer swelling agent.

9. A device as claimed in any of claims 1 to 6 wherein the hydrophilic gel comprises a thermally processable polyvinyl alcohol-based polymer.

10. A device as claimed in claim 9 wherein the polyvinyl alcohol-based polymer comprises
    a) from 16-20% by weight polyvinyl alcohol;
    b) from 10-30% by weight polar plasticizer; and
    c) from 1-6% by weight of the sodium salt of poly-2-acrylamido-2-methyl propanesulfonic acid.

## Revendications

1. Dispositif (10) pour incontinence adapté pour être inséré dans l'urètre et lorsqu'on le souhaite être retiré de celui-ci, qui comprend une portion (12) d'hydrogel hydrophile pouvant gonfler en présence des fluides corporels, ayant des dimensions correspondant pratiquement à celles de l'urètre, disposée autour d'une âme (14) souple non gonflable, ledit hydrogel lorsque le dispositif est inséré en utilisation dans le canal de l'urètre et exposé à des fluides, est adapté pour gonfler afin de constituer un joint de retenue contre les parois du canal de l'urètre en interdisant ou en contrôlant ainsi l'incontinence.

2. Dispositif suivant la revendication 1, caractérisé en ce que le gel hydrophile comprend au moins environ 40% en poids d'eau.

3. Dispositif selon l'une des revendications 1 ou 2, caractérisé en ce que la portion en hydrogel est constituée d'une bande disposée autour de l'âme.

4. Dispositif selon l'une quelconque des revendications 1 à 3, caractérisé en ce qu'il comporte en outre des moyens d'arrêt (20) à proximité d'une extrémité de celui-ci pour empêcher l'insertion du dispositif dans la vessie.

5. Dispositif selon la revendication 4, caractérisé en ce que les moyens d'arrêt (20) et la portion de gel gonflable (12) sont disposés à proximité des extrémités opposées du dispositif.

6. Dispositif suivant l'une quelconque des revendications 1 à 5, caractérisé en ce que l'âme comprend une extrémité évasée (28) adaptée pour permettre l'insertion du dispositif (10) dans l'urètre, l'extrémité opposée de celui-ci comprenant des moyens de retrait (33) pour permettre le retrait manuel du dispositif hors de l'urètre, l'hydrogel étant disposé de façon périphérique autour de l'âme (14) entre l'extrémité évasée et les moyens de retrait.

7. Dispositif selon l'une quelconque des revendications 1 à 6, caractérisé en ce que le gel hydrophile comporte :
   a) au moins environ 40% en poids d'eau;
   b) de 20 à 50% en poids de polyacrylamide;
   c) de 0 à 20% en poids de sel de sodium d'acide 2-acrylamido-2-méthyle propanesulfonique; et
   d) un agent réticulant.

8. Dispositif suivant l'une quelconque des revendications 1 à 6, caractérisé en ce que l'hydrogel comporte un réseau polymère interpénétrant (IPN) comprenant du polyacrylamide et un agent gonflant polymère.

9. Dispositif selon l'une quelconque des revendications 1 à 6, caractérisé en ce que le gel hydrophile comprend un polymère à base d'alcool polyvinylique thermotransformable.

10. Dispositif selon la revendication 9, caractérisé en ce que le polymère à base d'alcool polyvinylique comprend :
   a) de 16 à 20% en poids d'alcool polyvinylique;
   b) de 10 à 30% en poids de plastifiant polaire; et
   c) de 1 à 6% en poids de sel de sodium d'acide poly-2-acrylamido-2-méthyle propanesulfonique.

**Ansprüche**

1. Inkontinenzvorrichtung (10), die zum Einsetzen in die und, falls erwünscht, zum Herausnehmen aus der Harnröhre geeignet ist und die einen Teil aus hydrophilem, durch Körpermedien schwellfähigen Hydrogel (12) von im wesentlichen Harnröhrenabmessungen aufweist, der um einen im wesentlichen nicht schwellfähigen flexiblen Kern (14) herum angeordnet ist, wobei das Hydrogel, wenn die Vorrichtung in Gebrauch in Medien in dem Harnröhrentrakt eingebracht und diesen Medien ausgesetzt wird, schwellen kann, um für einen Zwangssitz gegen die Wandungen des Harnröhrentrakts zu sorgen und dadurch Inkontinenz zu verhindern oder zu kontrollieren.

2. Vorrichtung nach Anspruch 1, wobei das hydrophile Gel mindestens etwa 40 Gew.% Wasser aufweist.

3. Vorrichtung nach Anspruch 1 oder Anspruch 2, wobei der Hydrogelteil ein Band ist, das um den Umfang des Kerns herumgelegt ist.

4. Vorrichtung nach einem der Ansprüche 1 bis 3, ferner versehen mit einer benachbart ihrem einen Ende vorgesehenen Anschlaganordnung (20), um ein Einführen der Vorrichtung in die Blase zu verhindern.

5. Vorrichtung nach Anspruch 4, wobei die Anschlaganordnung (20) und der schwellfähige Gelteil (12) benachbart entgegengesetzten Enden der Vorrichtung angeordnet sind.

6. Vorrichtung nach einem der Ansprüche 1 bis 5, wobei der Kern ein verjüngtes Ende (28) aufweist, das geeignet ist, das Einführen der Vorrichtung (10) in die Harnröhre zu erlauben, und das entgegengesetzte Ende der Vorrichtung eine Herausnehmanordnung (33) aufweist, um ein Herausnehmen der Vorrichtung aus der Harnröhre von Hand zu erlauben, wobei das Hydrogel zwischen dem verjüngten Ende und der Herausnehmanordnung um den Umfang des Kerns (14) herumgelegt ist.

7. Vorrichtung nach einem der Ansprüche 1 bis 6, wobei das hydrophile Gel
   (a) mindestens etwa 40 Gew.% Wasser;
   (b) 20 bis 50 Gew.% Polyacrylamid;
   (c) 0 bis 20 Gew.% Natriumsalz der 2-Acrylamid-2-methylpropansulfonsäure; und
   (d) ein Vernetzungsmittel aufweist.

8. Vorrichtung nach einem der Ansprüche 1 bis 6, wobei das Hydrogel ein mit Polyacrylamid versehenes interpenetrierendes Polymernetzwerk (IPN) und ein Polymerquellungsmittel aufweist.

9. Vorrichtung nach einem der Ansprüche 1 bis 6, wobei das hydrophile Gel ein thermisch verarbeitbares Polymer auf der Basis von Polyvinylalkohol aufweist.

10. Vorrichtung nach Anspruch 9, wobei das Polymer auf der Basis von Polyvinylalkohol
   (a) 16 bis 20 Gew.% Polyvinylalkohol;
   (b) 10 bis 30 Gew.% polaren Weichmacher; und
   (c) 1 bis 6 Gew.% Natriumsalz der Poly-2-acrylamid-2-methylpropansulfonsäure aufweist.

Fig. 1

Fig. 3

Fig. 2

Fig. 4

Fig. 5a

Fig. 5b

EP 0 193 406 B1

Fig. 10

Fig. 7

Fig. 6

Fig. 8

Fig. 9a

Fig. 9b

18